# EUROPEAN PATENT SPECIFICATION

(11) **EP 3 934 431 B1**
(45) Date of publication and mention of the grant of the patent: **10.12.2025**
(21) Application number: 19718855.0
(22) Date of filing: 04.03.2019
(51) Int. Cl.: A01N 25/08, A01N 25/18, A01N 25/28, A01N 65/28, A01N 65/44, A61K 36/61, A61K 36/899, A01P 17/00

(54) **COMBINATIONS OF PLANT OILS AND THEIR USES**
KOMBINATIONEN VON PFLANZENÖLEN UND DEREN VERWENDUNGEN
COMBINAISONS D'HUILES VÉGÉTALES ET LEURS UTILISATIONS

(43) Date of publication of application: 12.01.2022
(73) Proprietor: Larus Pharma S.r.l., 20144 Milano (MI) (IT)
(72) Inventor: MODANESI, Roberto, 20144 Milano (MI) (IT)
(74) Representative: Gislon, Gabriele
(86) International application number: PCT/IB2019/051724
(87) International publication number: WO 2020/178617

(56) References cited:
- WO-A1-94/10842
- CN-A- 103 169 639
- CN-A- 105 052 925
- BEZAYIT SOLOMON ET AL: "Mosquito Repellent Actions of the Essential Oils of Cymbopogon citratus , Cymbopogon nardus and Eucalyptus citriodora : Evaluation and Formulation Studies", JOURNAL OF ESSENTIAL OIL-BEARING PLANTS, vol. 15, no. 5, 1 January 2012 (2012-01-01), IN, pages 766 - 773, XP055609893, ISSN: 0972-060X, DOI: 10.1080/0972060X.2012.10644118
- ASHISH UNIYAL ET AL: "Synergistic effect of effective oils against Aedes aegypti female mosquito, vector of dengue and chikungunya", INTERNATIONAL JOURNAL OF MOSQUITO RESEARCH, vol. 2, no. 4, 1 December 2015 (2015-12-01), pages 29 - 35, XP055609895, ISSN: 2348-5906, DOI: https://pdfs.semanticscholar.org/d3e5/5b69eedab6f4847243e331f62f14daf9cff2.pdf
- EVE ORGANIC BEAUTY: "Natural DIY Mosquito Repellent | Organic Beauty Recipes", ORGANIC BEAUTY RECIPES, 10 July 2011 (2011-07-10), pages 1 - 12, XP055609896, Retrieved from the Internet <URL:https://www.organic-beauty-recipes.com/natural-diy-mosquito-repellent/> [retrieved on 20190730]
- PASETA LORENA ET AL: "Encapsulation of essential oils in porous silica and MOFs for trichloroisocyanuric acid tablets used for water treatment in swimming pools", CHEMICAL ENGINEERING JOURNAL, ELSEVIER, AMSTERDAM, NL, vol. 292, 18 February 2016 (2016-02-18), pages 28 - 34, XP029448635, ISSN: 1385-8947, DOI: 10.1016/J.CEJ.2016.02.001

## Description

### Summary of the Invention

Object of the invention are novel combinations having insect-repelling, moth-repelling, anti-nausea activity and balsamic properties, said combinations being usable in micro-capsules and in combination with devices for the diffusion of aromas in the environment.

### Background of the Invention

Various devices and methods for the removal of mosquitoes and other annoying insects from people and environment by means of aromas have been known for a long time. CN105052925 discloses a mosquito repellent comprising p-methane-3,8-diol (PMD), deriving from Eucalyptus citriodora but is silent about citronella essential oil.

J. of essential oil-bearing plants, vol. 15(5), 2012, 766-773 discloses the repellent effects of a series of essential oil combination, including Eucalyptus citriodora with citronella essential oil (referred to as C. nardus) in a 1:1 ratio.

Int. J. of Mosquito Research, vol 2, n. 4, 2015, 29-35 discloses e repellent effects of a series of essential oil combination, including Eucalyptus citriodora (referred to as LMSN in table 1) with citronella essential oil (referred to as CITRO in table 1) in a 1:1 ratio, and shows that the effectiveness of said combination is considerably reduced just after 2 hours.

Organic Beauty Recipes, 2011, 1-12 discloses a mixture of Eucalyptus citriodora (referred to as Lemon eucalyptus) and citronella essential oil in a 1:1 ratio, dissolved in sunflower oil.

Chemical Engeneering Journal, vol 292, 2016, 28-34 discloses essential oils encapsulated in amorphous silica which do not show any controlled release. The document is silent about Eucalyptus citriodora.

A first type of known repelling devices provides for the release in the environment of toxic and/or unpleasant substances, such as for example pyrethrum and derivatives thereof, to the insects by means of combustion or vaporization. The main disadvantage of these devices is given by the fact that such substances are generally unpleasant and in some cases also moderately toxic for the people in the environment and further have low efficacy in open environments. A partial solution to these disadvantages is given by those devices consisting of repelling lotions or creams to be applied directly on the body. But these devices have the disadvantage that the repelling action of the compositions, once applied, have relatively short duration, and the chemical substances forming the repelling composition are contacted to the skin with possible toxic and/or allergic effects. A further disadvantage is given by the fact that it is necessary to apply the repelling composition on all the skin areas exposed or only slightly protected.

Patent Application WO94/10842 provided a first answer to obviate the drawbacks set forth above by means of a device providing an insect-repelling effect, thus avoiding the direct contact of the repelling composition with the skin. Such a device is essentially constituted by a container that is at least partially permeable to gases, in which micro-capsules containing some substances having an insect-repelling aroma are included. To carry out its effect, the device is preferably fixed, typically with some adhesives, to the body or the clothes of the subject who has to be protected from the insects or on a surface close to it. The insect-repelling agents contained in the micro-encapsulated compositions described in WO94/10842 are selected from essential oils of plants and synthesis composites, and in the experimental section of the document compositions containing citronella essential oils, basil essential oil and mixtures of lavender and citronella essential oils and geranium and basil essential oils are exemplified.

It has now been found that it is possible to boost the efficacy of the device described in WO94/10842 by using novel compositions having insect-repelling effect, in combination with novel particular technical measures relative to the micro-capsules and the materials used in the device structure.

Furthermore, it has been found that the devices adapted to contain micro-capsules, as that described in WO94/10842, can be used for the diffusion in the environment of other micro-encapsulated compositions, for example compositions having moth-repelling, anti-nausea and balsamic properties.

### Aims of the Invention

It is an aim of the invention providing novel combinations having insect-repelling properties that can be micro-encapsulated and used in combination with devices for the diffusion of aromas in the environment.

It is another aim of the invention providing novel combinations having moth-repelling, anti-nausea and balsamic properties that can be micro-encapsulated and used in combination with devices for the diffusion of aromas in the environment.

It is another aim of the invention providing novel compositions comprising the combinations of the invention together with one or more other components.

It is a further aim of the invention providing the use of the combinations or compositions of the invention in combination with devices for the diffusion of aromas in the environment.

### Brief Description of the Figures

Figure 1 and Figure 2 represent two cross sections of devices for the diffusion of aromas in the environment that can be used in combination with the combinations and compositions of the invention.
In Figure 3 is depicted a device suitable for the diffusion of the combinations and compositions of the invention, containing a plurality of containers of Figure 1.
Figure 4 depicts the apparatus used in a test for determining the efficacy of a composition representative of the invention.
Figures 5 and 6 depict the results of the test of Example 3.

### Description of the Invention

According to a first aspect, object of the invention is a combination consisting of Eucalyptus Citriodora essential oil in amounts ranging from 70% to 80% and Citronella essential oil in amounts ranging from 20% to 30%;
the total sum of compounds in the combination being equal to 100% by weight of the combination.

According to a preferred embodiment of the first aspect, object of the invention is a combination consisting of 75% Eucalyptus Citriodora essential oil + 25% Citronella essential oil.

Object of the invention is also the use of the combinations of the first aspect of the invention as insect-repelling agents.

The components of the combinations of the invention are all known in the art and can easily be found on the market.

For their use, the combinations of the invention are preferably used in compositions in which one or more inert materials are added, preferably silica, advantageously amorphous silica, and said compositions constitute a further object of the invention.

According to a preferred embodiment, the combinations or, preferably, the compositions of the invention are micro-encapsulated and are advantageously used in combination with devices for the diffusion in the environment. The micro-encapsulation of the combinations or, preferably, the compositions of the invention can be implemented by any know technique, such as for example spray-drying, extrusion, emulsification, coating, for example fluid bed coating or coacervation, the latter technique being preferred because it allows modulating the release of the substances contained in the micro-capsules.

It is necessary that the micro-capsules can be fractured by compression by means of light pressures, such as that exerted by the user's fingers. In fact, as it will be explained below, at the moment of use the user will break all or part of the micro-capsules to free its content in the environment.

The micro-capsules encapsulating the combinations or, preferably, the compositions of the invention, constitute a further object of the invention.

As stated, the compositions mentioned above, in addition to the combinations of the invention, can contain inert materials, preferably silica, advantageously amorphous silica. In fact, it has been unexpectedly found that by using the silica it is possible to obtain the same effect of loading and protection against moisture provided by the inert materials used in WO94/10842, while using a smaller quantity thereof. In fact, whereas in WO94/10842 inert materials were used in amounts ranging from 10% to 45% by weight, with respect to the total weight of the micro-capsule content, by using silica it is possible to limit such amount even below 5%, for example around 1.5-3%, preferably around 2% by weight, with respect to the total weight of the composition. This allows to load a higher amount of the combinations of the invention in the composition and thus in the micro-capsule and, accordingly, to obtain a boosting of the desired effect, with respect to what was possible by using the micro-capsules described in WO94/10842.

The micro-capsules can be made of any material suitable for releasing the agents contained in the compositions described above, for example gelatin or cellulose derivatives, the gelatin being preferred.

According to a preferred embodiment, the micro-capsules are made of the shell at 16-20% and by the combination, or preferably, the composition of the invention at 80-84%.

According to a preferred embodiment, the micro-capsules of the invention have a dimension between 400 and 1,100 microns.

With respect to the compositions described in WO94/10842, the micro-capsules having insect-repelling activity of the invention proved to be effective for a much longer period of time. In fact, whereas the experiments set forth in WO94/10842 demonstrated an active protection for 1.5 hours only, the representative compositions of the invention, as extensively set forth in the Experimental section, proved to be active for several hours, and up to 8 hours. This result is an important technical progress with respect to the teachings of the prior art.

As mentioned, the combinations and the compositions of the invention, in the micro-encapsulated form, are particularly suitable for the use in combination with aroma diffusers in the environment. Each device useful for the diffusion of aromas of the invention in the environment is a suitable one. Said device generally comprises at least one support layer and one layer made of permeable material between which the micro-capsules are placed.

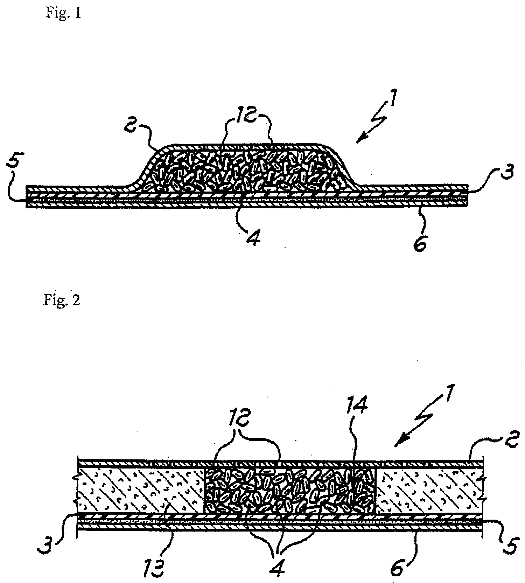

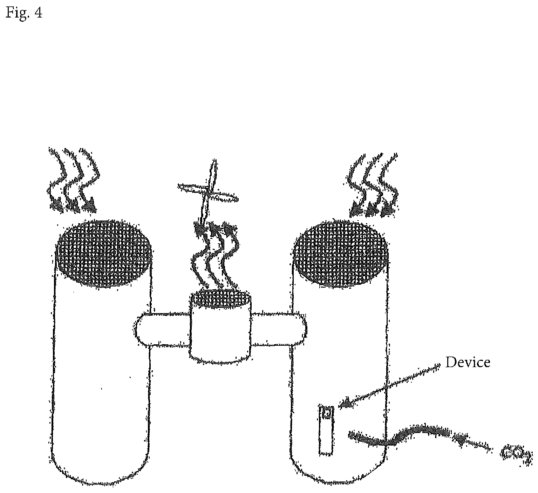

According to a preferred embodiment, the device for the diffusion of the combinations, or preferably of the compositions of the invention in the environment is the device described in WO94/10842. According to a particularly preferred form, said device is the one of the embodiments depicted in Figure 1 and Figure 4 of WO94/10842, to which reference is made for a detailed description, said figures being herein depicted as Figures 1 and 2 and briefly commented herein below.

As also set forth in document WO94/10842, a preferred device is equipped with a container 1 that, in Figure 1, is constituted by a material layer 2 permeable to the components of the compositions of the invention, that can be for example constituted by non-woven fabric, preferably constituted by a polypropylene resin, for example a polypropylene resin at 95% by weight or more and the remainder weight being conventional additives. The material 2 is coupled in a known way, for example by gluing or heat-sealing, with a material layer 3 that is impermeable to liquids instead and in general is constituted by plastic material, for example Moltopren, and preferably has thickness of 2-3 mm.

For a better permeability, the layer 2 can be provided with a plurality of holes 12. Between the two material layers a plurality of micro-capsules of the invention 4 is arranged.

According to an embodiment, below the layer 3, a layer 5 of self-adhesive material is arranged, preferably of the type that can be re-positioned, for removably fixing the device on the user (on the skin or clothes) or close to him. The adhesive layer 5 is in turn protected by one layer of silicon paper 6. The adhesive is preferably impermeable, hypoallergenic and dermatologically tested, high density polyethylene.

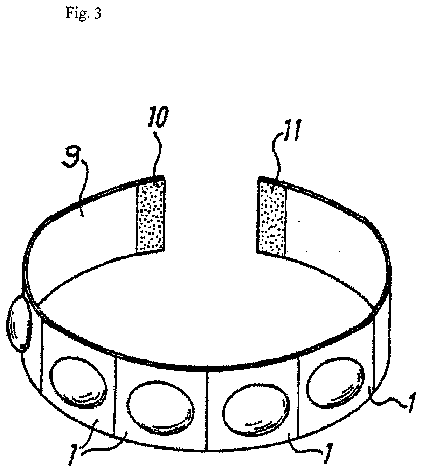

In another preferred embodiment, described in Figure 2 attached herein, the container 1 is provided with the same layers 2 to 6 of the embodiment of Figure 1, but in this case the upper layer 2 is coupled with the element 13 made of a porous material similar to a sponge, also permanently adhered to the layer 3. The material of the element 13 is advantageously made of expanded plastic material, such as polyurethane foam or polyethylene, known in the art and commercially available. The thickness of the element 13 is higher than that of the layers 2 and 3, and the element 13 is provided with a hole 14 which acts as a container for the micro-capsules of the invention 4. The devices usable according to the invention can contain only one container 1 or several containers 1 that can be activated by crashing the micro-capsules by means of a simple pressure by the user, simultaneously or sequentially over time, depending on the needs. By way of example, in Figure 3 is depicted another device suitable for the diffusion of the micro-encapsulated compositions of the invention, wherein a plurality of containers 1 according to Figure 1 are arranged in line by the adhesive layer 5 on a single stripe of silicon paper 9 that has the function of the layer 6 of Figure 1. Obviously other arrangements of the plurality of containers can be made.

According to other embodiments, still referring to the devices described above, the layers 5 and 6 can be omitted and the devices can be equipped with one or more means to be hung, such as hooks or the like. This solution can be for example useful for the diffusion of moth-repelling agents in the wardrobes.

If desired, the outer part of the material 2 can be customized with printings, also of high definition types.

Preferred devices according to the invention are those that are each able to house an amount of micro-capsules of the invention between about 55 and about 100 mg. Further details of the devices described above can be found in WO94/10842.

As mentioned, in addition to the devices set forth above, other devices can be used for the dispersion of the combinations and micro-encapsulated compositions of the invention.

The devices for the diffusion of aromas in the environments, comprising the micro-capsules of the invention, constitute a further object of the invention.

According to another of its aspects, object of the invention is a process for manufacturing the devices of the invention, comprising the following steps:
a. preparing the micro-capsules comprising the desired combination or composition, preferably by coacervation;
b. coupling the materials constituting the support of the device, except for the layer permeable to liquids;
c. inserting the micro-capsules on the support of the device; and
d. coating the support with the layer permeable to liquids.

According to an alternative embodiment, the micro-capsules of the invention can be applied on the permeable layer 2, and then the latter is coupled with the support of the device.

### Examples

Experimental tests aimed at demonstrating the efficacy of some representative compositions of the invention are described in the Examples reported herein.

### Example 1

### Study of the repelling efficacy against the mosquitoes of a device according to the invention, based on Citronella and Eucalyptus Citriodora essential oils according to the invention.

**Objective:** it is an purpose of the study to evaluate the repelling activity against the mosquitoes of a device comprising micro-capsules comprising Citronella and Eucalyptus Citriodora essential oils according to the invention, applied on the body.

### Material and Method

Living material:
- the test has been carried out with the female Aedes aegypti mosquitoes bred in laboratory. The original strain is the ORSTOM/OMS strain called Bora-bora,
- the females used for the test are 4 or 6 days old and are fasted for 24 hours (without meals based on blood) in order to increase their aggressiveness,
- the mouse to be protected is of the "nude" type and is kept in a cage on which the device is applied.

### Operating Method:

- the test is developed in a 60 cm cage for each side with aeration and a side door,
- the mouse is placed on a cube in a grid cage, thus making his movements impossible but allowing the bites of the mosquitoes,
- 25 to 45 females are introduced in the inner cage and the number of the insects leaning on the mouse and biting during a period of 15 minutes is recorded,
- the device is thus activated (crashing the micro-capsules) 2 minutes prior to letting the mosquitoes getting in.
- the number of insects that lean on the mouse and/or bite during a period of 15 minutes is recorded,
- this test is repeated three times in 3 different cages and with different mice.

### Variation:

- control test: for evaluating the efficacy of the device a control test without applying the device has been carried out,
- persistence: for evaluating the action duration the same procedure is applied by letting new groups of mosquitoes getting in again 2, 4, 6 hours after the activation of the device (without crashing again the micro-capsules) and the percentage of efficacy is recorded after 2, 4 and 6 hours.

### Test Results

Parameters: - number and percentage of mosquitoes leaned/biting
- 3 repeated tests: 3 cages with 3 different mice,
- 2 tests: control test without the device (A) + efficacy test with the device activated on the cage (B).

The results are shown in the following tables.

### A. Control test without the device

| | Mouse 1 38 mosquitoes in total | | Mouse 2 25 mosquitoes in total | | Mouse 3 29 mosquitoes in total | | Average | |
|---|---|---|---|---|---|---|---|---|
| Minutes | Number | % | Number | % | Number | % | Number | % |
| 2 | 8 | 21.1 | 4 | 16.0 | 5 | 17.2 | **5.7** | **18.1** |
| 4 | 17 | 44.7 | 11 | 44.0 | 9 | 31.0 | **12.3** | **39.9** |
| 6 | 18 | 47.4 | 16 | 64.0 | 14 | 48.3 | **16.0** | **53.2** |
| 8 | 19 | 50 | 15 | 60.0 | 18 | 62.1 | **17.3** | **57.4** |
| 10 | 21 | 55.3 | 14 | 56.0 | 17 | 58.6 | **17.3** | **56.6** |
| 12 | 20 | 52.6 | 13 | 52.0 | 15 | 51.7 | **16.0** | **52.1** |
| 14 | 22 | 57.9 | 16 | 64.0 | 16 | 55.2 | **18.0** | **59.0** |
| 15 | 23 | 60.5 | 17 | 68.0 | 17 | 58.6 | **19.0** | **62.4** |

### B. Efficacy test with the device

| | Mouse 1 38 mosquitoes in total | | Mouse 2 25 mosquitoes in total | | Mouse 3 29 mosquitoes in total | | Average | | Efficacy % |
|---|---|---|---|---|---|---|---|---|---|
| Minutes | Number | % | Number | % | Number | % | Number | % | |
| 2 | 1 | 2.4 | 0 | 0.0 | 1 | 3.1 | 0.7 | 1.9 | **89.5** |
| 4 | 1 | 2.4 | 1 | 3.4 | 1 | 3.1 | 1.0 | 3.0 | **92.5** |
| 6 | 2 | 4.9 | 0 | 0.0 | 1 | 3.1 | 1.0 | 2.7 | **94.9** |
| 8 | 2 | 4.9 | 0 | 0.0 | 0 | 0.0 | 0.7 | 1.6 | **97.2** |
| 10 | 2 | 4.9 | 1 | 3.4 | 1 | 3.1 | 1.3 | 3.8 | **93.3** |
| 12 | 0 | 0.0 | 2 | 6.9 | 1 | 3.1 | 1.0 | 3.3 | **93.7** |
| 14 | 1 | 2.4 | 2 | 6.9 | 0 | 0.0 | 1.0 | 3.1 | **94.7** |
| 15 | 1 | 2.4 | 2 | 6.9 | 1 | 3.1 | 1.3 | 4.2 | **93.3** |

Percentage results on % efficacy - Test repeated over time with the device

| | **15 min.** | **2 hours** | **4 hours** | **6 hours** |
|---|---|---|---|---|
| **Efficacy %** | **93.6** | **91** | **64** | **52** |

The table shows the average of the results of the repeated tests:
- the efficacy criterion is the percentage of repellency calculated by comparing the number of insects on the cage with or without the device,
- the efficacy of the patch on the cage is excellent (93%),
- the efficacy without further crashing of the micro-capsules remains 91% for the first 2 hours, then it decreases after 4 and 6 hours (respectively 64 and 52%).

Considering that with one crashing only not all of the micro-capsules have been broken and that with a second crashing other micro-capsules would release additional amounts of combination of the invention in the environment, it follows that crashing the micro-capsules at regular intervals provides excellent protection.

### Example 2

### Test on the efficacy of the device of the invention comprising a combination of Eucalyptus Citriodora and PMD (not according to the invention)

### Test Method

The following are used as animals for the test:
1. 300 - 400 yellow fever mosquitoes (Aedes aegypti) bred in laboratory, almost exclusively females. In a 40 x 40 x 40 cm breeding cage.
2. 300 - 400 malaria mosquitoes (Anofele gambiae) bred in laboratory, almost exclusively females, in a 40 x 40 x 40 cm breeding cage.

About 250 cm² on the lower arm of a volunteer are treated with about 2 ml of the composition mentioned above, for each type of mosquito. The non-treated area of the lower arm is protected up to the top of the arm with an anti-mosquitoes plastic protective sleeve and the hand is protected by an anti-mosquitoes woolen glove. The woolen glove permeable to air also serves as control area for the biting activity of the mosquitoes.

As a control for the behavior of the mosquito's population, each time a reference product is also tested.

Furthermore, the treated areas have not been touched during the test. The lower arm and the hand are kept in the cage of the mosquitoes once every hour for 10 minutes and is recorded the number of mosquitoes that, during the exposure period:
a) tries to bite the glove (positive control)
b) flies closer than 3 cm to the treated area
c) remains leaned on the treated area for more than 2 seconds and
d) bites the treated area and sucks the blood.

Evaluation and interpretation:
The efficacy of a substance is derived first of all by the ratio between the mosquitoes ready to bite and leaned on the glove (a) with respect to the remaining parameters (flying towards, leaning, biting).

Test interruption: the test is interrupted if the subject is bitten.

### First Test

### Mosquito type: Aedes aegypti

| Time after one application | Exposure time | Leaned on the glove | Mosquitoes flying over | Mosquitoes leaned | Number of bites |
|---|---|---|---|---|---|
| 1 hour | 10 min. | about 150 | 2 | 0 | 0 |
| 2 hours | 10 min. | about 150 | 5 | 1 | 0 |
| 3 hours | 10 min. | about 150 | 5 | 0 | 0 |
| 4 hours | 10 min. | about 150 | 3 | 1 | 0 |
| 5 hours | 10 min. | about 150 | 4 | 0 | 0 |
| 6 hours | 10 min. | about 100 | 7 | 3 | 0 |
| 7 hours | 10 min. | about 100 | 10 | 0 | 0 |
| 8 hours | 10 min. | about 100 | 4 | 3 | 0 |

### Mosquito type: anopheles gambiae

| Time after one application | Exposure time | Leaned on the glove | Mosquitoes flying over | Mosquitoes leaned | Number of bites |
|---|---|---|---|---|---|
| 1 hour | 10 min. | about 150 | 2 | 0 | 0 |
| 2 hours | 10 min. | about 150 | 3 | 1 | 0 |
| 3 hours | 10 min. | about 150 | 2 | 0 | 0 |
| 4 hours | 10 min. | about 100 | 5 | 0 | 0 |
| 5 hours | 10 min. | about 100 | 5 | 1 | 0 |
| 6 hours | 10 min. | about 100 | 4 | 2 | 0 |
| 7 hours | 10 min. | about 100 | 5 | 1 | 0 |
| 8 hours | 10 min. | about 100 | 7 | 2 | 0 |

### Test Results

The composition of the invention was shown to have no side effects and demonstrated an optimal repelling effect against Aedes aegypti and Anopheles gambiae, which lasted up to more than eight hours.

The established effect is comparable to that of many products with synthetic substances on the market.

### Repelling effect until the first bite, protection against the bites = average

| | Aedes aegypti result | Anopheles gambiae result | Conditions required for the commercial repelling agents |
|---|---|---|---|
| Repelling effect Maximum number of mosquitoes | ≤ 10 | ≤ 10 | ≤ 10 |
| Duration of the protection against the bites | > 6.5 hours | > 6.0 hours | 4.0 hours |

### Example 3

### Test on the efficacy of the composition comprising 2-undecanone and Eucalyptus citriodora (not according to the invention)

The device used for the test is depicted in Figure 1. The two big cylindrical chambers on the left and right were 40 cm in height and 25 cm in diameter and have been built with a thin sheet of transparent plastic. The shelves were covered with a BugBed 123 (Green Thumb Group Inc., Downer's Grave, Illinois) plastic screen, whereas the bottoms have been attached to plastic trays with tape. The central cylinder from which the mosquitoes were released in the device was built with the same solid plastic material and was 5.5 cm in height and 4.5 cm in diameter. The upper part was covered with the BugBed 123 screen and the bottom with a solid plastic material as previously described.

A PVC tube (11 cm length and 1.5 cm outer diameter) extended between the left and right cylinders for 40 x 25 cm and was opened at each end. The adult mosquitoes had unlimited access to all of the inner surfaces of the device. A fan was used to direct the flow of air as depicted in Figure 4. The device was housed in the incubator, and all of the experiments were carried out at 27°C ± 3°C, 65% relative humidity (±4%).

The selected test is with three ways. When the adult mosquitoes were introduced in the central chamber, they could remain in the central cylinders or could move in the vertical left or right cylinders during the course of the test. Yellow fever mosquitoes (Aedes aegypti) and Asian tiger mosquitoes (Aedes albopictus) used for these tests were three or four weeks old, female, fed with water and sugar. Some carbon dioxide at room temperature and/or the composition of the invention have been introduced to the bottom of the right vertical cylinder, the closest possible to the center of the chamber. The carbon dioxide was released from a small plastic container with distilled water laid at the bottom of the cylinder. A small plastic tube carried the gas from a compressed gas cylinder to the bottom of the water, producing bubbles. The carbon dioxide bubbles have been used for monitoring the gas flow, and a continuous gas flow that was highly attractive for the mosquitoes, as detected by a high percentage of movement in the right cylinder, has been selected. The composition of the invention has been introduced in the right vertical cylinder at 5.5 cm from the bottom.

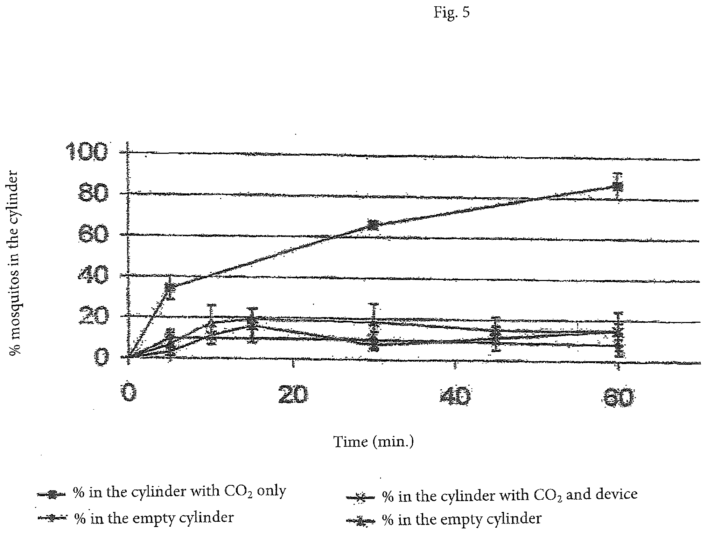

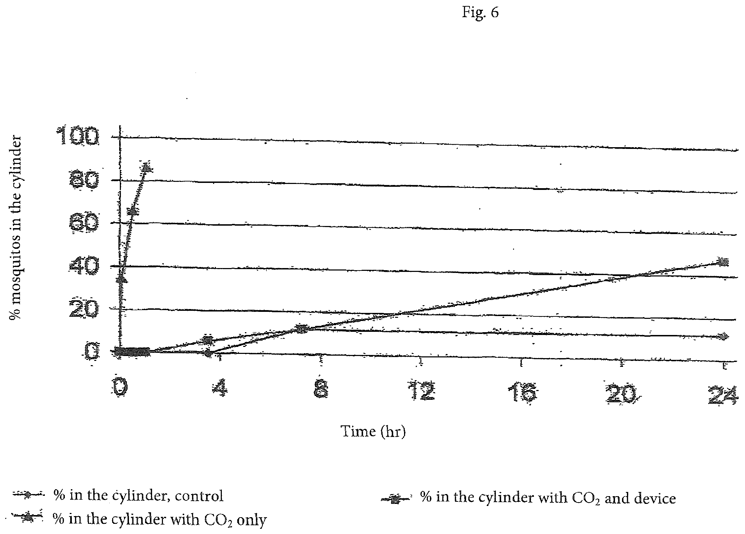

The test results are reported in Figures 5 and 6. 30 mosquitoes and two devices containing a composition with an active ingredient concentration of 10% and 30% by weight, with respect to the total weight of the composition, have been used.

### Test Results

### Experiment 1

Two different bio-tests have been carried out by using the Aedes aegypti mosquitoes: Experiment (a): carbon dioxide in the right vertical cylinder vs. absence of carbon dioxide and absence of the composition of the invention in the left vertical cylinder. Experiment (b): carbon dioxide and composition of the invention at 10% in the right vertical cylinder vs. absence of carbon dioxide and absence of the composition of the invention in the left vertical cylinder.

The results of the two experiments are depicted in Figure 5.

For the experiment (a) carbon dioxide was a great attraction and, after 60 minutes, more than 80% of the mosquitoes leaned in the right cylinder with respect to less than 20% of the mosquitoes that were found in the left cylinder. On the contrary, in the experiment (b), less than 20% of the mosquitoes moved in the right cylinder containing the device and most of the mosquitoes remained in the central chamber in which they have been initially introduced.

These experiments have been repeated in triplicate and the error margin in Figure 5 is ± 1 standard error of the mean.

The conclusion drawn from the experiment 1 is that, in the test conditions, the device of the invention at 10% has been effective in the removal of mosquitoes from carbon dioxide.

### Experiment 2

A single experiment has been carried out by using Aedes albopictus mosquitoes: carbon dioxide and composition of the invention at 30% in the right vertical cylinder (R) vs. absence of carbon dioxide and absence of the composition of the invention in the left vertical cylinder (L).

This bio-test has been carried out in duplicate, once for 60 minutes and once for 24 hours. For the 60 minutes test, the following results have been obtained, expressed as percentage of mosquitoes in each cylinder: 0/13% (R/L) at 5 minutes; 0/17% at 10 minutes; 0/17% at 15 minutes; 0/17% at 20 minutes; 0/17% at 25 minutes; 0/26% at 30 minutes; 0/26% at 45 minutes; 0/30% at 50 minutes; 0/35% at 60 minutes.

Figure 6 depicts the results of the 24 hours test. After 3.5 hours, no mosquito has been found in the right cylinder containing carbon dioxide and the composition of the invention at 30%. After 7.5 hours the percentage of mosquitoes in the right cylinder was 5% and after 24 hours 45%.

The percentage data of mosquitoes in the cylinder with carbon dioxide but without the composition has been taken from Figure 5 and added as a positive control.

The conclusion drawn from the experiment 2 is that, in the test conditions, the 30% composition of the invention has been effective in the removal of mosquitoes from carbon dioxide for several hours.

## Claims

1. A combination consisting of Eucalyptus Citriodora essential oil in amounts ranging from 70% to 80% and Citronella essential oil in amounts ranging from 20% to 30%; the total sum of compounds in the combination being equal to 100% by weight of the combination.

2. The combination according to claim 1 wherein said 75% Eucalyptus citriodora essential oil is combined with 25% Citronella essential oil.

3. Use of a combination according to claim 1 or 2 as insect-repelling agent.

4. A composition consisting of the combination according to anyone of claims 1 or 2 and at least one inert material

5. The composition according to claim 4, **characterized in that** said inert material is silica.

6. Micro-capsules encapsulating a combination according to anyone of claims 1 or 2 or the composition according to anyone of claim 4 or 5.

7. Use of the micro-capsules according to claim 6 in diffusers of aromas in the environment.

8. A diffuser of aromas in the environment comprising the micro-capsules according to claim 6.

## Patentansprüche

1. Eine Kombination bestehend aus ätherischem Öl von Eucalyptus Citriodora in Mengen von 70 % bis 80 % und ätherischem Öl von Citronella in Mengen von 20 % bis 30 %, wobei die Gesamtsumme der Bestandteile in der Kombination 100 % des Gewichtes der Kombination beträgt.

2. Die Kombination nach Anspruch 1, wobei 75 % ätherisches Öl von Eucalyptus Citriodora mit 25 % ätherischem Öl von Citronella kombiniert sind.

3. Verwendung einer Kombination nach Anspruch 1 oder 2 als insektenabweisendes Mittel.

4. Eine Zusammensetzung bestehend aus der Kombination nach einem der Ansprüche 1 oder 2 und mindestens einem inerten Material.

5. Die Zusammensetzung nach Anspruch 4, **dadurch gekennzeichnet, dass** das inerte Material Kieselsäure ist.

6. Mikrokapseln, die eine Kombination nach einem der Ansprüche 1 oder 2 oder die Zusammensetzung nach einem der Ansprüche 4 oder 5 einkapseln.

7. Verwendung der Mikrokapseln nach Anspruch 6 in Diffusoren von Aromen in der Umgebung.

8. Ein Diffusor von Aromen in der Umgebung, umfassend die Mikrokapseln nach Anspruch 6.

## Revendications

1. Combinaison constituée d'huile essentielle d'Eucalyptus Citriodora en quantités allant de 70 % à 80 % et d'huile essentielle de Citronnelle en quantités allant de 20 % à 30 % ; la somme totale des composés de la combinaison étant égale à 100 % en poids de la combinaison.

2. Combinaison selon la revendication 1 dans laquelle l'huile essentielle d'Eucalyptus citriodora à 75 % est combinée avec l'huile essentielle de Citronnelle à 25 %.

3. Utilisation d'une combinaison selon la revendication 1 ou 2 comme agent répulsif des insectes.

4. Composition constituée de la combinaison selon l'une quelconque des revendications 1 ou 2 et d'au moins un matériau inerte.

5. Composition selon la revendication 4, **caractérisée en ce que** ledit matériau inerte est de la silice.

6. Micro-capsules encapsulant une combinaison selon l'une quelconque des revendications 1 ou 2 ou la composition selon l'une quelconque des revendications 4 ou 5.

7. Utilisation des micro-capsules selon la revendication 6 dans des diffuseurs d'arômes dans l'environnement.

8. Diffuseur d'arômes dans l'environnement comprenant les micro-capsules selon la revendication 6.
